# EUROPÄISCHE PATENTSCHRIFT

(11) **EP 4 011 335 B1**
(45) Veröffentlichungstag und Bekanntmachung des Hinweises auf die Patenterteilung: **27.03.2024**
(21) Anmeldenummer: 21210348.5
(22) Anmeldetag: 25.11.2021
(51) Int. Cl.: A61F 5/01, A61F 5/37

(54) **SCHULTERGELENKORTHESE ZUR STABILISIERUNG DER SCHULTER EINES PATIENTEN**
SHOULDER ORTHOSIS FOR STABILIZING THE SHOULDER OF A PATIENT
ORTHÈSE D'ARTICULATION D'ÉPAULE DESTINÉE À LA STABILISATION DE L'ÉPAULE D'UN PATIENT

(30) Priorität: 08.12.2020 DE 102020132550
(43) Veröffentlichungstag der Anmeldung: 15.06.2022
(73) Patentinhaber: Bort GmbH, 71384 Weinstadt-Benzach (DE)
(72) Erfinder: Roscher, Philipp, 09117 Chemnitz (DE); Niemann, Tobias, 01159 Dresden (DE); Barchen, Andreas, 72622 Nürtingen (DE)
(74) Vertreter: Thum, Bernhard

(56) Entgegenhaltungen:
- US-A- 5 403 268
- US-A1- 2020 214 869
- US-B1- 10 660 780
- US-B2- 10 646 366

## Beschreibung

Die vorliegende Erfindung betrifft eine Schultergelenkorthese zur Stabilisierung der Schulter eines Patienten mit den Merkmalen des Anspruchs 1. Vorteilhafte Weiterbildungen der Erfindung sind in den abhängigen Ansprüchen 2-13 definiert. Die Schultergelenkorthese umfasst eine Armbandage zum Anbringen an einem Arm des Patienten, sodass die Armbandage im Tragezustand der Schultergelenkorthese den Oberarm des Patienten zumindest teilweise umgibt, eine Schulterbandage zum Anbringen an einer Schulter des Patienten, sodass die Schulterbandage im Tragezustand der Schultergelenkorthese die Schulter des Patienten zumindest teilweise bedeckt, und ein Seilzugsystem, das die Armbandage mit der Schulterbandage über ein umgelenktes Zugseil verbindet. Das Seilzugsystem umfasst wenigstens ein Seilumlenkelement und wenigstens ein Seilspannelement, wobei sich das Zugseil beidseits des Seilumlenkelements mit zwei Zugseilabschnitten zugwirksam erstreckt und das Zugseil mit dem Seilspannelement in seiner zugwirksamen Länge derart veränderbar ist, dass die Positionierung der Armbandage zu der Schulterbandage einstellbar ist.

Schultergelenkorthesen werden verwendet, um nach einer Verletzung die Schulter eines Patienten zu stützen und je nach Indikation vollständig oder teilweise ruhig zu stellen. Es hat sich jedoch gezeigt, dass geringfügige und kontrollierte Bewegungen des Armes für die Heilung der verletzten Schulter förderlich sein können. Zudem ermöglicht eine gewisse Beweglichkeit des Armes dem Patienten, ohne Hilfestellung alltägliche Bewegungen in eingeschränktem Maße durchzuführen. Dennoch sollte das verletzte Schultergelenk soweit stabilisiert sein, dass durch die Schultergelenkorthese gelenkschädigende oder luxationsfördernde Bewegungen des Oberarms weitestgehend verhindert werden.

Aus dem Stand der Technik sind zweiteilige Schultergelenkorthesen bekannt, bei denen der eine Teil an die Anatomie eines menschlichen Armes und der andere Teil an die Anatomie einer menschlichen Schulter angepasst ist. Aus dem Gebrauchsmuster DE 92 15 341 U1 ist beispielsweise eine zwei Schalen umfassende Schultergelenkorthese bekannt, bei der die Schalen über ein Torsionsgelenk miteinander verbunden und über ein Gurtsystem an dem Patienten befestigbar sind. Nachteilig ist bei dieser Schultergelenkorthese jedoch, dass ihre Stützwirkung nicht individuell einstellbar bzw. an den Patienten anpassbar ist. Folglich kann die Stützung in Hinblick auf die tatsächliche Verletzung bzw. den Lauf der Genesung zu gering bzw. zu stark sein. Ferner schränkt die Schultergelenkorthese die Bewegung des Armes nach vorne und hinten kaum ein, was ruckartige Bewegungen des Patienten ermöglicht, die für die Heilung hinderlich sein können.

Ferner ist eine zweiteilige Schultergelenkorthese aus dem veröffentlichten US-Patent US 10,646,366 B2 bekannt, welches die Merkmale des Oberbegriffs des Anspruchs 1 offenbart. Das Ausmaß der Stützung der Schulter kann über ein Seilzugsystem eingestellt werden, wobei eine Drehung einer Ratsche die Zugseilspannung erhöht bzw. reduziert. Auf diese Weise kann der Patient ohne die Hilfe einer weiteren Person den Grad der Stützung anpassen, den die Schultergelenkorthese für die verletzte Schulter vorsieht. Jedoch schränkt diese Schultergelenkorthese die Bewegung des Armes derart stark ein, dass ein Pendeln des Oberarmes nur schwer möglich ist. Gerade solche Pendelbewegung des Oberarms sollten aber für einen positiven Heilungsverlauf möglich sein.

Eine weitere Schultergelenkorthese ist aus der US 2020/0214869 A1 bekannt. Bei dieser Schultergelenkorthese sind mehrere Zugseile vorgesehen, die sich von einer Schulterbandage zu einer Armbandage erstrecken und fest an der Armbandage fixiert sind.

Weiterhin ist aus der US 10 660 780 B1 eine Schultergelenkorthese bekannt, die mittels verschnürten Bändern zwischen der Schulter- und der Armbandage eine Fixierung des Armes um 360 Grad bereitstellt.

Es ist daher Aufgabe der vorliegenden Erfindung, eine Schultergelenkorthese bereitzustellen, deren Stützwirkung an den Grad der Schulterverletzung und die jeweils erforderliche Behandlung anpassbar ist. Darüber hinaus soll die Schultergelenkorthese die Beweglichkeit des Armes der verletzten Schulter soweit ermöglichen, dass die Heilung nicht beeinträchtigt wird und geringe Bewegungen des Alltags zugelassen werden.

Diese Aufgabe wird mit einer Schultergelenkorthese mit den Merkmalen des Anspruchs 1 gelöst. Weiterbildungen der erfindungsgemäßen Schultergelenkorthese finden sich in den abhängigen Ansprüchen wieder.

Bei der erfindungsgemäßen Schultergelenkorthese ist vorgesehen, dass diese gemäß der eingangs bezeichneten Art ausgebildet ist und ferner das Seilumlenkelement derart an der Schulterbandage befestigt ist, dass im Tragezustand der Schultergelenkorthese das Seilumlenkelement auf der Schulter des Patienten angeordnet ist.

Die Verwendung des Seilzugsystems als verbindendes Element zwischen der Schulterbandage und der Armbandage ermöglicht eine kraftsparende Reponierung des Humeruskopfes (Oberarmkopfes) in das Schultergelenk. Die zur Reponierung erforderliche Kraft kann abhängig von der Indikation der Schulterverletzung und dem Fortschreiten der Heilung der Schulterverletzung unterschiedlich sein. An diese unterschiedlichen Anforderungen an die Stabilisierung des Schultergelenks kann die erfindungsgemäße Schultergelenkorthese angepasst werden. Zu diesem Zweck wird die zugwirksame Länge und folglich die Position der beiden Bandagen, also der Schulterbandage und der Armbandage, zueinander verändert.

Die zugwirksame Länge des Zugseils kann definiert sein als die Länge eines Zugseilabschnitts, der zwischen der Schulterbandage und der Armbandage verläuft. Die zugwirksame Länge kann also kürzer sein als die Gesamtlänge des Zugseils. Zum Beispiel bewirkt eine Verkürzung der zugwirksamen Länge des Zugseils, dass sich die Armbandage in Richtung der Schulterbandage bewegt, wodurch der Arm des Patienten Richtung der Schulter des Patienten gezogen wird. Umgekehrt bewirkt eine Verlängerung der zugwirksamen Länge des Zugseils, dass sich die Armbandage von der Schulterbandage weg bewegt, wodurch die Stützung der Schulter des Patienten verringert wird. Es kann also die Stützwirkung, also die Stabilisierung des Schultergelenks, an den Grad der Schulterverletzung und die jeweils erforderliche Behandlung angepasst werden.

Erfindungsgemäß ist das Seilumlenkelement derart an der Schulterbandage befestigt, dass im Tragezustand der Schultergelenkorthese das Seilumlenkelement auf der Schulter des Patienten angeordnet ist. Diese erfindungsgemäße Positionierung des Seilumlenkelements ermöglicht es dem Patienten, dass er seinen Arm in die Flexion, also Beugung, und Extension, also Streckung, bewegen kann. Die Bewegungsfreiheit des Patienten im Tragezustand der Schultergelenkorthese ist folglich verbessert gegenüber herkömmlichen Schultergelenkorthesen. Trotz verbesserter Bewegungsfreit wird die Bewegung nur soweit zugelassen, dass die zur Heilung des Schultergelenks benötigte Stabilisierung gewährleistet ist. Dennoch sind beim Tragen der Schultergelenkorthese geringe Bewegungen des Alltags für den Patienten möglich und er ist weniger auf Hilfe durch andere Personen angewiesen. Die Positionierung des Seilumlenkelements auf der Schulter ist folglich auf die Anatomie des menschlichen Körpers angepasst, wodurch die Heilung der Schulterverletzung verbessert ist.

Genauer spezifiziert kann das Seilumlenkelement auf der Schulter bedeuten, dass das Seilumlenkelement im Tragezustand der Schultergelenkorthese kranial, also zu dem Kopf des Patienten weisend, angeordnet ist. Zudem kann die Bezeichnung "auf der Schulter" einem Bereich zugeordnet sein, der sich zwischen einem Schulterdach und einem Kopf des Patienten erstreckt. Auch kann die Bezeichnung "auf der Schulter" einem Bereich zugeordnet sein, der sich benachbart einem Schlüsselbein oder/und einem Schultereckgelenk oder/und einem Acromion (Schulterdach) des Patienten befindet.

Ferner ist erfindungsgemäß vorgesehen, dass wenigstens ein weiteres Seilumlenkelement an der Armbandage angebracht ist, wobei das Zugseil ausgehend von der Schulterbandage durch das wenigstens eine weitere Seilumlenkelement hindurchgeführt ist, sodass ein weiterer Zugseilabschnitt die Armbandage über das wenigstens eine weitere Seilumlenkelement mit der Schulterbandage verbindet. Auf diese Weise wird eine gewisse Bewegungsfreiheit des Oberarmes für Pendelbewegungen ermöglicht, jedoch gelenkschädigende oder luxationsfördernde Bewegungen des Oberarms noch besser verhindert. Durch eine konträre Anordnung der zueinander liegenden Umlenkpunkte von Schulterbandage zur Armbandage wird zudem eine außenrotatiorische Bewegung entgegen einer innenrotatorisch-luxationsfördernden Bewegung bewirkt.

Gemäß einer Weiterbildung der Erfindung kann die Armbandage noch ein weiteres Seilumlenkelement aufweisen, wobei ein erstes der zwei Seilumlenkelemente im Tragezustand der Schultergelenkorthese auf der Vorderseite der Armbandage angeordnet ist und ein zweites der zwei Seilumlenkelemente im Tragezustand der Schultergelenkorthese auf der Rückseite der Armbandage angeordnet ist. Die Schultergelenkorthese kann also wenigstens zwei oder genau zwei Seilumlenkelemente umfassen. Diese ermöglichen eine gewisse Bewegungsfreiheit des Oberarmes für Pendelbewegungen, verhindern jedoch gelenkschädigende oder luxationsfördernde Bewegungen des Oberarms weitestgehend.

Die Bezeichnungen "Vorderseite" und "Rückseite" können im Tragezustand der Schultergelenkorthese in Bezug auf den Körper des Patienten als "ventral" bzw. "dorsal" verstanden werden.

Gemäß der Erfindung ist das wenigstens ein Seilumlenkelement, das an der Armbandage angeordnet ist, von der Armbandage zerstörungsfrei lösbar. Alternativ ist das Zugseil außer Eingriff mit dem wenigstens einem Seilumlenkelement bringbar, das an der Armbandage angeordnet ist. Es können auch beide Seilumlenkelemente, die an der Armbandage angeordnet sind, entsprechend einer der zwei vorstehenden alternativen Ausführungen ausgebildet sein.

Beispielsweise ist das wenigstens eine Seilumlenkelement, das an der Armbandage angeordnet ist, mit einem zweiteiligen Verbindungselement an der Armbandage befestigt. Ein erster Teil des Verbindungselements kann mit der Armbandage fest verbunden sein, z.B. vernietet sein, und ein zweiter Teil des Verbindungselements kann mit dem Zugseil fest verbunden und in Eingriff mit dem ersten Teil bringbar sein. Beispielsweise sind beide Teile zusammensteckbar. Es versteht sich, dass die zwei Teile des Verbindungselements wiederholt verbunden und wieder getrennt werden können.

Unabhängig von der gewählten Umsetzung einer lösbaren Verbindung zwischen dem Zugseil und der Armbandage, ermöglicht dies ein bequemes und separates Anlegen der Armbandage und der Schulterbandage an den Patienten, ohne störenden Einfluss durch das Zugseil. Sind beide Bandagen an den Patienten angelegt, können die Seilumlenkelemente an der Armbandage angebracht oder das Zugseil wieder in Eingriff mit dem wenigstens einen Seilumlenkelement an der Armbandage gebracht werden.

Eine weitere Verbesserung der Stabilisierung des Schultergelenks kann dadurch erzielt werden, dass die Schulterbandage an ihrer Vorderseite wenigstens ein weiteres Seilumlenkelement umfasst, das im Tragezustand brustseitig unterhalb des Schlüsselbeins des Patienten angeordnet sein kann. Dieses Seilumlenkelement an der Vorderseite der Schulterbandage kann zur definierten Führung des Zugseils eingerichtet sein, wobei das Zugseil ausgehend von der Armbandage durch das Seilumlenkelement an der Vorderseite der Schulterbandage und zu dem Seilspannelement geführt sein kann.

Ferner kann eine weitere Verbesserung der Stabilisierung des Schultergelenks dadurch erzielt werden, dass die Schulterbandage an ihrer Rückseite wenigstens ein weiteres Seilumlenkelement umfasst, das im Tragezustand im Bereich des Schulterblattes oder des Oberarmknochenkopfs des Patienten angeordnet sein kann. Bevorzugt kann die Schulterbandage an ihrer Rückseite zwei Seilumlenkelemente umfassen, wobei eines näher an dem Oberarmknochenkopf des Patienten und eines näher an der Wirbelsäule des Patienten angeordnet ist.

Mittels der Bereitstellung der weiteren Seilumlenkelemente an der Vorderseite oder/und der Rückseite der Schulterbandage kann während einer Bewegung des Oberarms die Schulter optimal gestützt werden, da hier der Verlauf des Zugseils sehr gut an die Schulterbewegung angepasst ist. Zudem kann dadurch die durch das Zugseil ausgeübte Zugkraft über die Schulterbandage verteilt werden und folglich die Stütz- und Stabilisierungseigenschaften der Schultergelenkorthese verbessert werden.

Gemäß einer Weiterbildung der Erfindung kann das im Tragezustand auf der Schulter des Patienten angeordnete Seilumlenkelement mittels eines Verbindungselements mit der Schulterbandage verbunden sein, das eine vorzugsweise elastische Bewegung des Zugseils gegenüber der Schulterbandage zulässt. Alternativ oder zusätzlich kann das wenigstens eine der Seilumlenkelemente an der Armbandage mittels eines weiteren Verbindungselements angeordnet sein, das zumindest teilweise elastisch ist und eine vorzugsweise elastische Bewegung des Zugseils gegenüber der Armbandage zulässt.

Eine geringfügige elastische Bewegung des Zugseils gegenüber einer oder beider der Bandagen erhöht die Dynamik und den Tragekomfort der Schultergelenkorthese. Gemäß einer Ausführungsvariante der Erfindung ist vorgesehen, dass das Seilspannelement im Tragezustand der Schultergelenkorthese brustbeinnah an der Schulterbandage angeordnet ist. D.h. das Seilspannelement ist bevorzugt ventral angeordnet. Auf diese Weise kann es der Patient im Tragezustand gut greifen. Zudem ist das Seilspannelement bevorzugt außerhalb des Schulterbereichs der zu stützenden Schulter angeordnet. Auf diese Weise befindet sich das Seilspannelement außerhalb des Behandlungsbereichs, also außerhalb eines Bereichs, wo der Patient gegebenenfalls Schmerzen verspürt.

Das Seilspannelement ist dazu eingerichtet, die zugwirksame Länge des Zugseils zu verändern. Beispielsweise verändert das Seilspannelement die zugwirksame Länge des Zugseils mittels einer translatorischen oder einer rotatorischen Bewegung des Zugseils. Eine translatorische Bewegung kann mittels eines Greifzügels erzielt werden, der an verschiedenen Positionen an der Schulterbandage befestigbar sein kann. Zur Befestigung können zu verbindende Flächen der Schulterbandage und des Seilspannelements mit aneinanderhaftenden Materialen ausgeführt sein. Beispielsweise kann Mikroklett an wenigstens einer der zu verbindenden Flächen vorhanden sein. Eine rotatorische Bewegung kann mittels eines Drehsystems erfolgen. Hier wird zum Beispiel an das Drehsystem Boa^{®} gedacht, bei welchem ein Zugseilabschnitt in dem Drehsystem aufgenommen ist. Je nach Drehung des Drehsystems verändert sich die Länge des Zugseilabschnitts, der in dem Drehsystem aufgenommen ist. Das Drehsystem kann ortsfest mit der Schulterbandage verbunden sein.

Eine definierte Führung des Zugseils an den Bandagen der Schultergelenkorthese kann erzielt werden, indem das Zugseil zumindest abschnittsweise oder/und bei wenigstens einem Teil der Seilumlenkelemente innerhalb wenigstens einer Zugseilführung verläuft, die mit der Schulterbandage verbunden ist. Die Schultergelenkorthese kann mehrere Zugseilführungen umfassen. Wenigstens eines der Seilumlenkelemente kann eine ihm zugeordnete Zugseilführung umfassen.

Die Zugseilführung ist beispielsweise ein Kunststoffröhrchen. Die wenigstens eine Zugseilführung kann zwischen dem Seilumlenkelement auf der Schulter und dem wenigstens einem Seilumlenkelement an der Armbandage angeordnet sein. Die wenigstens eine Zugseilführung kann sich zwischen der Vorderseite und der Rückseite der Schulterbandage erstrecken und ist vorzugsweise im Tragezustand der Schultergelenkorthese auf der Schulter des Patienten angeordnet.

Gemäß einer Weiterbildung der Erfindung ist ein Trägermaterial der Schulterbandage oder/und der Armbandage ein unelastisches oder ein flexibles, elastisches Stoffmaterial. Dies ermöglicht ein angenehmes und komfortables Tragen der Schultergelenkorthese. Die Schulterbandage umfasst beispielsweise ein Obermaterial aus einem Material, auf dem ein Klettverschluss befestigbar ist, z.B. klettbares Velour. Alternativ oder zusätzlich sind Ränder der Schulterbandage oder/und der Armbandage bevorzugt mittels eines Einfassbandes umfasst.

Zwischen dem Trägermaterial der Schulterbandage oder/und der Armbandage und dem wenigstens einem Seilumlenkelement kann ein Verstärkungsmaterial, z.B. aus Kunststoff, angeordnet sein. Auf diese Weise können Druckstellen durch das wenigstens eine Seilumlenkelement vermieden werden. Es versteht sich, dass zwischen allen Seilumlenkelementen und der zugeordneten Bandage das Verstärkungsmaterial angeordnet sein kann. Das Seilumlenkelement, z.B. die Zugseilführung, kann mit dem Verstärkungsmaterial vernäht sein.

In einer Ausführungsform der Erfindung kann die Zugseilführung in Form eines Kunststoffröhrchens ausgeführt sein, das mittels Kunststofftulpen mit dem Verstärkungsmaterial verbunden ist, z.B. vernäht, vernietet oder verschraubt ist.

Das wenigstens eine Seilumlenkelement, beispielsweise die Zugseiführung, kann zumindest einen entsprechenden Zugseilabschnitt des Zugseils vollständig umgeben. Auf diese Weise kann ein unerwünschtes Herausrutschen des Zugseils aus dem Seilumlenkelement vermieden werden.

Das wenigstens eine Seilumlenkelement kann die Position des Zugseils an der Schulterbandage bzw. der Armbandage definieren, jedoch eine Bewegung des Zugseils relativ zu der Längserstreckung des Zugseils zulassen.

Im Folgenden wird eine bevorzugte Ausführungsform der Armbandage beschrieben. Gemäß dieser umfasst die Armbandage zwei Öffnungen zum Durchführen eines Armes des Patienten. Alternativ oder zusätzlich kann die Armbandage wenigstens ein Verschlusselement umfassen, das dazu ausgelegt ist, die Armbandage an einem Arm des Patienten zu fixieren. Das Verschlusselement kann ein Gurtband sein. Auch kann die Armbandage zwei Verschlusselemente, z.B. zwei Gurtbänder, umfassen, die dazu ausgelegt sind, die Armbandage an einem Arm des Patienten zu fixieren.

Die Armbandage kann als eine Ellenbogenbandage ausgebildet sein, die einen Ellenbogen des Patienten im Tragezustand zumindest teilweise bedeckt. In der Beuge des Ellenbogengelenks kann die Armbandage eine Materialaussparung aufweisen, wodurch unerwünschte Reibstellen an der Haut des Patienten vermeidbar sind. Bei einer Armbandage, die als Ellenbogenbandage ausgebildet ist, kann ein Verschlusselement distal und ein weiteres Verschlusselement proximal des Ellenbogengelenks angeordnet sein.

Im Folgenden wird eine bevorzugte Ausführungsform der Schulterbandage beschrieben. Die Schulterbandage kann dazu ausgelegt sein, im Tragezustand der Schultergelenkorthese die der zu behandelnden Schulter benachbarte Brust sowie den benachbarten Achselbereich des Patienten zumindest teilweise zu bedecken und unter der kontralateralen Achsel des Patienten durchgeführt zu werden. Die kontralaterale Achsel ist der Schulter des Patienten benachbart, die von der Schultergelenkorthese nicht stabilisiert wird. Die Befestigung unter der kontralateralen Achsel kann als Achselgurt bezeichnet werden.

Ein Verschlussmechanismus der Schulterbandage kann wenigstens ein Verschlusselement umfassen, das beispielsweise im Tragezustand nahe der kontralateralen Brust angeordnet ist. Das Verschlusselement kann eine Schnalle sein. Die Schnalle kann mittels eines Gurtbandes an der Schulterbandage befestigt sein, wobei die Position des Gurtbandes gegenüber der Schnalle den Innenumfang, d.h. den Brustumfang, der Schulterbandage festlegt. Ferner kann die Schulterbandage eine Lasche umfassen, die im Tragezustand nahe der Achsel an der zu stabilisierenden Schulter angeordnet sein kann. Die Lasche ist derart angeordnet, dass sie eine Anpassung der Schulterbandage an den Übergangsbereich von dem Oberarm zur Schulter des Patienten ermöglicht. Mittels der Lasche kann ein Hindurchführen des Armes durch einen umfänglich verschlossenen Armansatz vermieden werden. Die Schulterbandage kann folglich sehr einfach an den Patienten angebracht werden.

Gemäß einer Weiterbildung der Erfindung umfasst das Seilzugsystem genau ein Zugseil, das offen oder geschlossen ist. Ferner kann das Zugseil umlaufend sein. Das Zugseil kann auch offen sein und Endabschnitte der zugwirksamen Länge des Zugseils können an dem Seilspannelement zusammengeführt sein. Das Zugseil kann ein Stahlseil sein.

Im Folgenden werden verschiedene Ausführungsformen der Erfindung beispielhaft anhand der beiliegenden Figuren erläutert. Es stellen dar:
- Figuren 1a-d: perspektivische Ansichten einer Schultergelenkorthese im Tragezustand der Schultergelenkorthese gemäß einer ersten Ausführungsform der Erfindung;
- Figur 2: eine perspektivische Ansicht einer Schultergelenkorthese im Tragezustand gemäß einer zweiten Ausführungsform der Erfindung;
- Figur 3: eine Draufsicht auf eine erste Seite eines Schnittmusters der Schultergelenkorthese gemäß der zweiten Ausführungsform der Erfindung;
- Figuren 4a-b: eine jeweilige Draufsicht auf Ausschnitte einer zweiten Seite des Schnittmusters von Figur 3 in einem verbundenen und einem gelösten Zustand; und
- Figur 5: einen die Schulter und die Brust umfassenden Ausschnitt der Anatomie des menschlichen Körpers.
Figurenbeschreibung

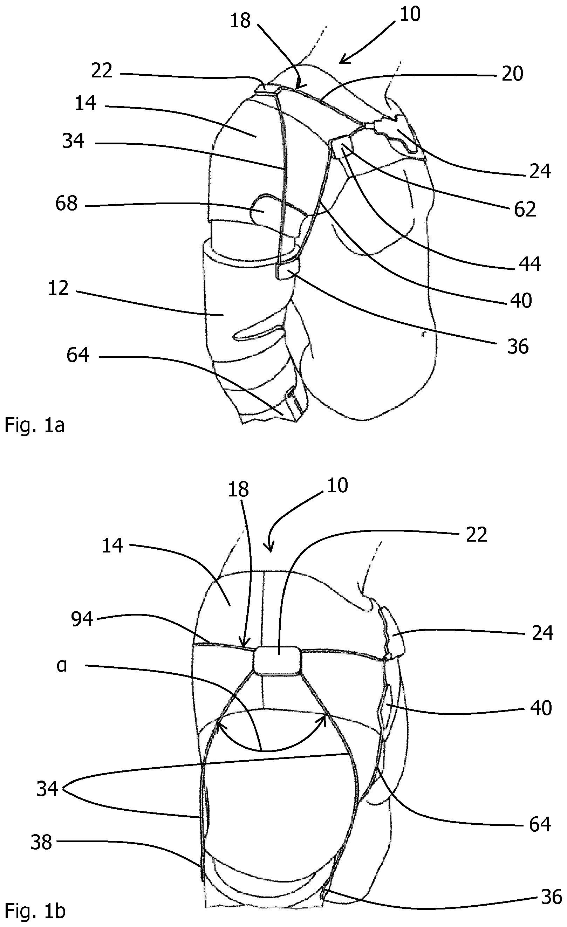

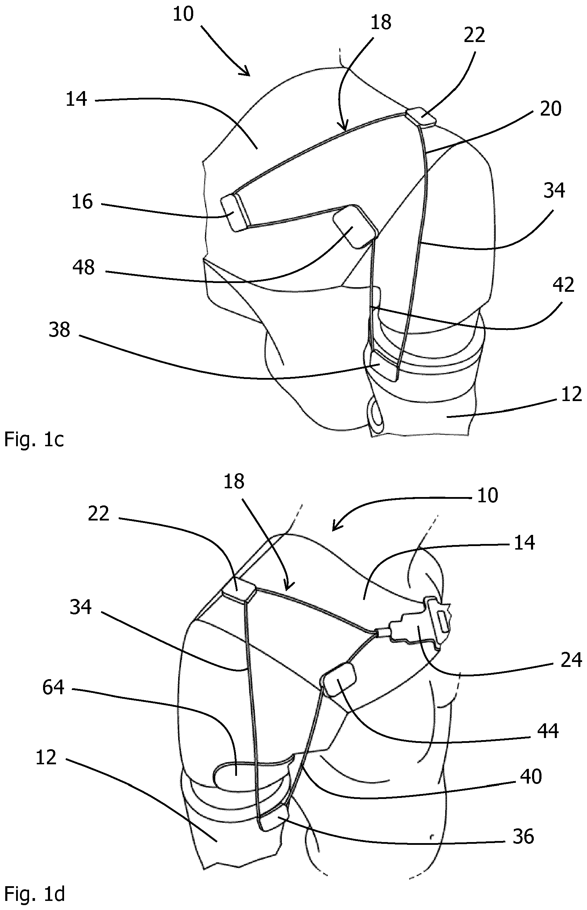

In Figuren 1a-d ist eine Ausführungsform einer erfindungsgemäßen Schultergelenkorthese in verschiedenen Perspektiven gezeigt und allgemein mit 10 bezeichnet. Die Schultergelenkorthese 10 ist im Tragezustand dargestellt, also in einem Zustand, in dem diese an einem menschlichen Körper angebracht ist. Die Schultergelenkorthese 10 umfasst zwei Bandagen 12, 14, deren Trägermaterialien 16 nicht miteinander verbunden sind. Eine der zwei Bandagen ist eine Armbandage 12 zum Anbringen an einem Arm des Patienten. In der dargestellten Ausführungsform ist die Armbandage 12 als Ellenbogenbandage 12 ausgebildet. Die andere der zwei Bandagen ist eine Schulterbandage 14 zum Anbringen an einer Schulter des Patienten.

Die zwei Bandagen 12, 14 sind über ein Seilzugsystem 18 verbunden, das ein umgelenktes Zugseil 20 umfasst. Das Zugseil 20 steht in Eingriff mit wenigstens einem Seilumlenkelement 22 und einem Seilspannelement 24. Das Seilspannelement 24 ist dazu eingerichtet, eine zugwirksame Länge des Zugseils 20 zu verändern. Als zugwirksame Länge kann diejenige Länge des Zugseils 20 verstanden werden, die wirksam die Positionierung der Armbandage 12 in Bezug auf die Schulterbandage 14 beeinflusst.

Das Seilspannelement 24 ist gemäß der in den Figuren 1a-d dargestellten Ausführungsform positionsveränderlich an der Schulterbandage 14 angebracht. Beispielsweise kann das Seilspannelement 24 an zwei oder mehr verschiedenen Positionen an der Schulterbandage 14 angebracht werden. Eine erste Position stellt beispielsweise eine größere zugwirksame Länge des Zugseils 20 bereit, sodass die Bandagen 12, 14 weiter voneinander beabstandet sind. Eine zweite Position stellt beispielsweise eine im Vergleich zur ersten Position kürzere zugwirksame Länge des Zugseils 20 bereit, sodass die Bandagen 12, 14 im Vergleich zur ersten Position näher beieinander sind.

In einer alternativen - nicht dargestellten - Ausführungsform kann das Seilspannelement ein Drehsystem, beispielsweise ein Boa^{®}-System, umfassen, das ortsfest mit der Schulterbandage 14 verbunden ist. Eine Drehung des Drehsystems kann die in dem Drehsystem aufgenommene Seillänge verändern und folglich die zugwirksame Länge des Zugseils 20 verkürzen bzw. verlängern.

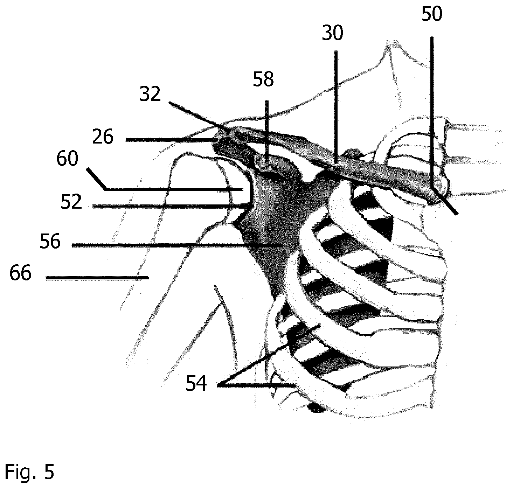

Das Seilumlenkelement 22 auf der Schulter ist im Tragezustand der Schultergelenkorthese auf der Schulter des Patienten angeordnet. Beschreibt man die Anordnung des Seilumlenkelements 22 auf der Schulter in Bezug auf die in Figur 5 dargestellte Anatomie des menschlichen Körpers, dann ist dieses im Tragezustand in einem Bereich angeordnet, der sich ausgehend von einem Schulterdach 26 kranial erstreckt, sich also z.B. zwischen dem Schulterdach 26 und einem Kopf 28 des Patienten erstreckt. Auch ist dieses im Tragezustand benachbart einem Schlüsselbein 30 oder/und einem Schultereckgelenk 32 oder/und dem Schulterdach 26 des Patienten angeordnet. Eine Anordnung des Seilumlenkelements 22 auf der Schulter kann eine Anordnung ausschließen, bei der das Seilumlenkelements 22in Bezug auf das Schultereckgelenk 32 distal angeordnet ist.

Das Zugseil 20 erstreckt sich von dem Seilumlenkelement 22 auf der Schulter mit einem Zugseilabschnitt 34 zu der Armbandage 12. Ein Winkel α zwischen dem Zugseilabschnitt 34, der sich beidseits des Seilumlenkelements 22 auf der Schulter erstreckt, beträgt weniger als 90 Grad, bevorzugt weniger als 85 Grad, ferner bevorzugt weniger als 80 Grad. In einer besonders bevorzugten Ausführung beträgt der Winkel α zwischen 65 Grad und 75 Grad, insbesondere 70 Grad.

Die Armbandage 12 weist ein erstes Seilumlenkelement 36 an der Vorderseite der Armbandage 12 und ein zweites Seilumlenkelement 38 an der Rückseite der Armbandage 12 auf. Das Zugseil 20 ist somit ausgehend von der Schulterbandage durch das erste und das zweite Seilumlenkelement 36, 38 hindurchgeführt und erstreckt sich dann ausgehend von dem ersten und dem zweiten Seilumlenkelement 36, 38 über zwei weitere Zugseilabschnitte 40, 42 zurück zu der Schulterbandage 14. Auf diese Weise sind die zwei Bandagen 12, 14 über den Zugseilabschnitt 34 sowie über die zwei weiteren Zugseilabschnitte 40, 42 miteinander verbunden.

Ferner weist die Schulterbandage 14 weitere Seilumlenkelemente 44, 46, 48 auf, von denen eines 44 auf der Vorderseite und zwei 46, 48 auf der Rückseite der Schulterbandage 14 angeordnet sind. Bezogen auf die in Figur 5 dargestellte Anatomie des menschlichen Körpers ist das Seilumlenkelement 44 an der Vorderseite der Schulterbandage 14 zwischen einem Brustbein 50 und einem Armansatz 52, bevorzugt benachbart der Rippen 54, angeordnet. Eines der Seilumlenkelemente 46 auf der Rückseite der Schulterbandage 14 ist im Tragezustand im Bereich des Schulterblattes 56 oder/und dessen Fortsatzes 58 angeordnet. Das andere 48 der zwei Seilumlenkelemente auf der Rückseite der Schulterbandage 14 ist im Tragezustand benachbart des Oberarmkopfes 60 angeordnet. Wenigstens eines der zwei Seilumlenkelemente 46, 48 an der Rückseite der Schulterbandage ändert eine Verlaufsrichtung des Zugseils 20 um mehr als 120 Grad, bevorzugt mehr als 140 Grad, ferner bevorzugt mehr als 160 Grad. In einer besonders bevorzugten Ausführung beträgt die Änderung der Verlaufsrichtung des Zugseils 20 zwischen 160 Grad und 180 Grad, insbesondere 170 Grad.

In den Figuren 1a-d erkennt man, dass die Seilumlenkelemente 22, 36, 38, 44, 46, 48 zumindest einen Abschnitt des Zugseils 20 vollständig umgeben. Ferner sind die Seilumlenkelemente zumindest teilweise auf dem einem Material der jeweiligen Bandage 12, 14 mittels eines an das Material genieteten Polsterkissens 62 befestigt.

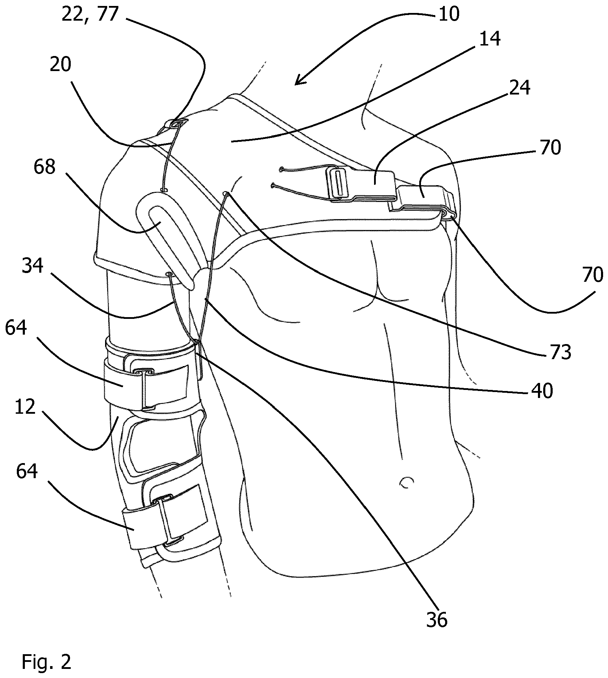

Für eine definierte Positionierung der Armbandage 12 an dem Arm des Patienten weist diese wenigstens ein Verschlusselement 64 auf, sodass die Armbandage 12 mittels des Verschlusselements 64 umlaufend um den Arm des Patienten herum fixiert ist, wie in Figur 2 ersichtlich ist. Die Armbandage 12 ist im Tragezustand derart positioniert, dass diese im Bereich eines Oberarmes 66 des Patienten von der Schulterbandage 14 beabstandet ist.

Die Schulterbandage 14 weist für eine definierte Positionierung dieser an dem Patienten eine Befestigung im Bereich der der zu stabilisierenden Schulter benachbarten Achsel und eine Befestigung unter der kontralateralen Achsel des Patienten auf. Die Befestigung im Bereich der zu stabilisierenden Schulter kann ein ausgebildeter Armansatz sein oder/und eine Lasche 68 sein, die unter der Achsel benachbart der zu stabilisierenden Schulter durchgeführt ist, wie in Figuren 1a und 2 ersichtlich. Die Befestigung unter der kontralateralen Achsel des Patienten kann ein Verschlusselement 70, beispielsweise eine Schnalle sein. Das Verschlusselement 70 kann mit einem an der Schulterbandage 14 befestigten Gurtband derart verbunden sein, dass der Umfang der Schulterbandage 14 veränderbar ist.

Im Folgenden wird in Bezug auf die Figuren 2 bis 4a-b eine zweite Ausführungsform der Schultergelenkorthese 10 beschrieben, die sich von der ersten Ausführungsform im Wesentlichen durch die Ausführung der Seilumlenkelemente und der Materialzusammensetzung der zwei Bandagen 12, 14 unterscheidet. Im Folgenden werden daher lediglich diejenigen Merkmale in Bezug auf die zweite Ausführungsform beschrieben, die sich von denjenigen der ersten Ausführungsform unterscheiden.

Figur 2 zeigt die Schultergelenkorthese 10 im Tragezustand. Die Armbandage 12 ist mittels zwei Verschlusselementen 64 an dem Arm des Patienten angebracht und die Schulterbandage 14 ist mittels der Lasche 68 und dem Verschlusselement 70 an dem Patienten angebracht. Das Seilspannelement 24 ist an der Schulterbandage 14 lösbar fixiert. Das Zugseil 20 ist lediglich in Bereichen sichtbar, in denen es durch ein Obermaterial 72 auf eine Oberseite der Schulterbandage geführt ist. Hierzu sind Öffnungen 73 in dem Obermaterial 72 vorhanden sein, die z.B. durch Ösen verstärkt sein können.

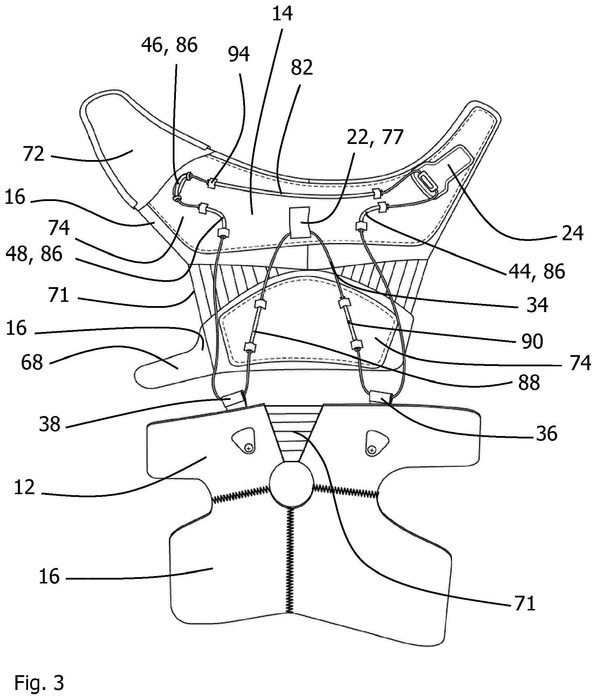

Figur 3 zeigt ein Schnittmuster der Schultergelenkorthese 10 gemäß der zweiten Ausführungsform, wobei die Verschlusselemente 64, 70 nicht dargestellt sind. Bereiche, bei denen entsprechende Abschnitte des Schnittmusters zusammengenäht werden, sind mit Linien 71 angedeutet. Im oberen Teil der Figur 3 ist das Schnittmuster der Schulterbandage 14 dargestellt. Figur 3 zeigt ferner den Materialaufbau der Schulterbandage 14 mit dem Trägermaterial 16, einem Verstärkungsmaterial 74, beispielsweise aus Kunststoff, und dem Obermaterial 72, was lediglich teilweise angedeutet ist. Die Armbandage 12 kann den gleichen Materialaufbau aufweisen oder lediglich das Trägermaterial 16 und das Obermaterial 72 umfassen. Ferner können sich die Materialien des Trägermaterials 16, gegebenenfalls des Verstärkungsmaterials 74, und des Obermaterials 72 der beiden Bandagen 12, 14 voneinander unterscheiden oder zumindest teilweise gleich sein. Das Trägermaterial 16 kann ein unelastisches oder kann ein flexibles, elastisches Stoffmaterial sein. Es kann beispielsweise aus einem textilen Abstandsgewirke oder aus einem anderen kaschierten Material hergestellt sein.

Mittig der oberen Schnittdarstellung von Figur 3 ist das Seilumlenkelement 22 ersichtlich, das im Tragezustand auf der Schulter des Patienten angeordnet ist. Das Seilumlenkelement 22 auf der Schulter des Patienten ist in der dargestellten Ausführungsform ein Band 77, das das Zugseil 20 umgibt und mittels welchem dieses geführt ist. Das Band 77 kann beispielsweise elastisch sein, also eine Bewegung des Zugseils 20 relativ zu einem Befestigungspunkt des Seilumlenkelements 22 auf der Schulter des Patienten ermöglichen. Es versteht sich, dass eine solche Bewegung relativ gering ist, also lediglich eine Bewegung von wenigen Millimetern, beispielsweise weniger als 5 mm, ermöglicht. Das Seilumlenkelement 22 auf der Schulter ist zumindest an einer Stelle mit der Schulterbandage 14, beispielsweise mit dem Verstärkungsmaterial 74 oder/und dem Trägermaterial 16, fest verbunden. Das Verbindungselement, mittels welchem das Seilumlenkelement 22 auf der Schulter an der Schulterbandage 14 befestigt ist, kann vernietet oder vernäht sein.

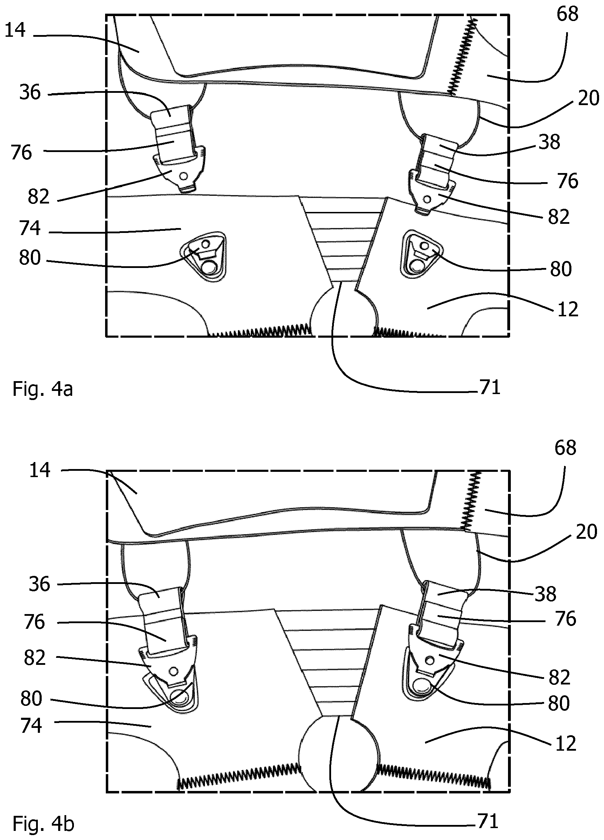

Das erste und das zweite Seilumlenkelement 36, 38 an der Armbandage 12 sind in ähnlicher Weise wie das vorstehend beschriebene Seilumlenkelement 22 auf der Schulter ausgebildet, wie in den Figuren 4a-b dargestellt. Jedoch unterscheiden sich die Seilumlenkelemente 36, 38 an der Armbandage 12 dadurch, dass diese an der Armbandage 12 lösbar befestigt sind. Dies wird durch ein zweiteiliges Verbindungselement 78 erzielt, von dem ein erster Teil 80 fest mit der Armbandage 12 verbunden ist und ein zweiter Teil 82 mit dem ersten Teil 80 verbindbar ist. Figur 4a zeigt das Verbindungselement 78 in einem verbundenen Zustand und Figur 4b in einem gelösten Zustand. Das erste Teil 80 des Verbindungselements 78 ist mit dem Obermaterial 72 oder/und dem Verstärkungsmaterial 74 vernietet. Das Seilumlenkelement 36 bzw. 38 an der Armbandage ist als Band 76, beispielsweise elastisches Band, ausgebildet und mit dem zweiten Teil 82 des Verbindungselements 78 fest verbunden. Wie in Figuren 4a-b ersichtlich, ist das Zugseil 20 in Eingriff mit dem Seilumlenkelement 36 bzw. 38 an der Armbandage 12 und wird durch dieses geführt. Das Seilumlenkelement 36 bzw. 38 umfasst zur Führung des Zugseils 20 eine Zugseilführung 84, beispielsweise in Form eines Kunststoffröhrchens.

Erneut unter Bezugnahme auf Figur 3 wird nun die Zugseilführung im Bereich der Schulterbandage 14 näher beschrieben. Das Seilumlenkelement 44 an der Vorderseite der Schulterbandage 14 und die Seilumlenkelemente 46, 48 an der Rückseite der Schulterbandage 14 sind in Form von gebogenen Zugseilführungen 86 ausgebildet.

Ferner umfasst die Schulterbandage 14 zusätzliche Zugseilführungen 88, 90, 92, die einen definierten Verlauf von Zugseilabschnitten 34 ermöglichen, die zwischen Seilumlenkelementen verlaufen. Der Zugseilabschnitt 34 zwischen den Seilumlenkelementen 36, 38 an der Armbandage und dem Seilumlenkelement 22 auf der Schulter wird durch wenigstens eine oder wie in dem dargestellten Ausführungsbeispiel durch zwei Zugseilführungen 88, 90 geführt, die im Tragezustand im Bereich des Oberarmes, beispielsweise benachbart des Oberarmkopfes 60, angeordnet sind. Der Zugseilabschnitt 34 befindet sich zwischen dem Seilumlenkelement 46 an der Rückseite der Schulterbandage 14 und dem Seilspannelement 24.

Bei der Zugseilführung 86-92 kann es sich um ein Kunststoffröhrchen handeln, dessen longitudinalen hohlen Innenraum das Zugseil 20 durchtritt. Der Innendurchmesser der Zugseilführung 86-92 ist bevorzugt lediglich geringfügig, z.B. maximal doppelt so groß, wie der Außendurchmesser des Zugseils 20. Im Allgemeinen können wenigstens 20 %, besser wenigstens 25 %, noch besser wenigstens 30 % der zugwirksamen Länge des Zugseils innerhalb mehrerer Zugseilführungen 86-92 verlaufen. In einer besonders bevorzugten Ausführung verlaufen zwischen 30 % und 45 %, insbesondere 35 % der zugwirksamen Länge des Zugseils 20 innerhalb mehrerer Zugseilführungen 86-92.

Die Zugseilführung 86-92 kann mit dem Verstärkungsmaterial 74 verbunden sein. Beispielsweise kann die Zugseilführung 86-92 mittels Kunststofftulpen 94 mit der Schulterbandage 14 bzw. dem Verstärkungsmaterial 74 davon verbunden sein, beispielsweise vernäht sein.

Das Obermaterial 72 der Schulterbandage 14 deckt zumindest einige der Seilumlenkelemente 44, 46, 48 oder/und der Zugseilführungen 86-92 ab. Das Verbindungselement des Seilumlenkelements 22 auf der Schulter ist von dem Obermaterial 72 der Schulterbandage 14 bedeckt, also zwischen dem Trägermaterial 16 und dem Obermaterial 72 der Schulterbandage 14 angeordnet. Die Verbindungselemente 80, 82 der Seilumlenkelemente 36, 38 an der Armbandage 12 sind auf dem Obermaterial 72 der Armbandage 12 befestigt.

Wie in Figur 2 ersichtlich, ist das Seilspannelement 24 im Tragezustand der Schultergelenkorthese 10 im Bereich des Brustbeins 50 des Patienten angeordnet. In der in Figur 2 dargestellten Ausführungsform kann das Seilspannelement 24 mittels eines Mikrokletts an der Schulterbandage 14, genauer gesagt an dessen Obermaterial 72, befestigt werden. Je nachdem, wo die Befestigung des Seilspannelements 24 auf der Schulterbandage 14 erfolgt, ändert sich die zugwirksame Länge des Zugseils 20. Würde das Seilspannelement 24 weiter in Richtung der kontralateralen Schulter befestigt, würde sich die zugwirksame Länge des Zugseils 20 vergrößern. Würde hingegen das Seilspannelement 24 näher an der zu stabilisierenden Schulter befestigt, würde sich die zugwirksame Länge des Zugseils 20 verringern.

## Patentansprüche

1. Schultergelenkorthese (10) zur Stabilisierung der Schulter eines menschlichen Patienten, umfassend:
- eine Armbandage (12) zum Anbringen an einem Arm des Patienten, sodass die Armbandage (12) im Tragezustand der Schultergelenkorthese den Oberarm des Patienten zumindest teilweise umgibt,
- eine Schulterbandage (14) zum Anbringen an einer Schulter des Patienten, sodass die Schulterbandage (14) im Tragezustand der Schultergelenkorthese die Schulter des Patienten zumindest teilweise bedeckt, und
- ein Seilzugsystem (18), das die Armbandage (12) mit der Schulterbandage (14) über ein umgelenktes Zugseil (20) verbindet,
wobei das Seilzugsystem (18) wenigstens ein Seilumlenkelement (22) und wenigstens ein Seilspannelement (24) umfasst, wobei sich das Zugseil (20) beidseits des Seilumlenkelements (22) mit zwei Zugseilabschnitten (34) zugwirksam erstreckt und das Zugseil (20) mit dem Seilspannelement (24) in seiner zugwirksamen Länge derart veränderbar ist, dass die Positionierung der Armbandage (12) zu der Schulterbandage (14) einstellbar ist,
wobei
das Seilumlenkelement (22) derart an der Schulterbandage (14) befestigt ist, dass im Tragezustand der Schultergelenkorthese (10) das Seilumlenkelement (22) auf der Schulter des Patienten angeordnet ist, wobei - wenigstens ein weiteres Seilumlenkelement (36, 38) an der Armbandage (12) angebracht ist, wobei das Zugseil (20) ausgehend von der Schulterbandage (14) durch das wenigstens eine weitere Seilumlenkelement (36, 38) hindurchgeführt ist, sodass ein weiterer Zugseilabschnitt (40) die Armbandage (12) über das wenigstens eine weitere Seilumlenkelement (36, 38) mit der Schulterbandage (14) verbindet, **dadurch gekennzeichnet,**
- **dass** das wenigstens eine weitere Seilumlenkelement (36, 38), das an der Armbandage (12) angeordnet ist, von der Armbandage (12) zerstörungsfrei abnehmbar ist, oder dass das Zugseil (20) außer Eingriff mit dem wenigstens einen weiteren Seilumlenkelement (36, 38) bringbar ist, das an der Armbandage (12) angeordnet ist.

2. Schultergelenkorthese (10) nach Anspruch 1, **dadurch gekennzeichnet, dass** das wenigstens eine weitere Seilumlenkelement (36, 38), das an der Armbandage angeordnet ist, aus zwei Seilumlenkelementen (36, 38) besteht, wobei ein erstes der zwei Seilumlenkelemente (36) im Tragezustand der Schultergelenkorthese (10) auf der Vorderseite der Armbandage (12) angeordnet ist und ein zweites der zwei Seilumlenkelemente (38) im Tragezustand der Schultergelenkorthese (10) auf der Rückseite der Armbandage (12) angeordnet ist.

3. Schultergelenkorthese (10) nach einem der vorangehenden Ansprüche, **dadurch gekennzeichnet, dass** die Schulterbandage (14) an ihrer Vorderseite wenigstens ein weiteres Seilumlenkelement (44) umfasst, das im Tragezustand brustseitig unterhalb des Schlüsselbeins (30) des Patienten angeordnet ist.

4. Schultergelenkorthese (10) nach einem der vorangehenden Ansprüche, **dadurch gekennzeichnet, dass** die Schulterbandage (14) an ihrer Rückseite wenigstens ein weiteres Seilumlenkelement (46, 48) umfasst, das im Tragezustand im Bereich des Schulterblattes (56) oder des Oberarmknochenkopfs (60) des Patienten angeordnet ist, wobei die Schulterbandage (14) an ihrer Rückseite bevorzugt zwei Seilumlenkelemente (46, 48) umfasst, wobei eines näher an dem Oberarmknochenkopf (60) des Patienten und eines näher an der Wirbelsäule des Patienten angeordnet ist.

5. Schultergelenkorthese (10) nach einem der vorangehenden Ansprüche, **dadurch gekennzeichnet, dass** das im Tragezustand auf der Schulter des Patienten angeordnete Seilumlenkelement (22) mittels eines Verbindungselements (77) mit der Schulterbandage (14) verbunden ist, das eine, vorzugsweise elastische, Bewegung des Zugseils (20) gegenüber der Schulterbandage (14) zulässt.

6. Schultergelenkorthese (10) nach einem der vorangehenden Ansprüche, **dadurch gekennzeichnet, dass** das Seilspannelement (24) im Tragezustand der Schultergelenkorthese (10) brustbeinnah an der Schulterbandage (14) angeordnet ist.

7. Schultergelenkorthese (10) nach einem der vorangehenden Ansprüche, **dadurch gekennzeichnet, dass** das Seilspannelement (24) mittels einer translatorischen oder einer rotatorischen Bewegung des Zugseils (20) die zugwirksame Länge des Zugseils (20) verändert.

8. Schultergelenkorthese (10) nach einem der vorangehenden Ansprüche, **dadurch gekennzeichnet, dass** das Zugseil (20) zumindest abschnittsweise oder/und bei wenigstens einem Teil der Seilumlenkelemente (22, 36, 38,44, 46, 48) innerhalb einer Zugseilführung (86-92) verläuft, die mit der Schulterbandage (14) verbunden ist.

9. Schultergelenkorthese (10) nach einem der vorhergehenden Ansprüche, **dadurch gekennzeichnet, dass** ein Trägermaterial (16) der Schulterbandage (14) oder/und der Armbandage (12) ein flexibles, elastisches Stoffmaterial ist, wobei vorzugsweise zwischen dem Trägermaterial (16) der Schulterbandage (14) oder/und der Armbandage (12) unddemwenigstens einem der Seilumlenkelemente (22, 36, 38,44, 46, 48) ein Verstärkungsmaterial (74), z.B. aus Kunststoff, angeordnet ist.

10. Schultergelenkorthese (10) nach einem der vorhergehenden Ansprüche, wobei wenigstens eines der Seilumlenkelemente (22, 36, 38,44, 46, 48) zumindest einen entsprechenden Zugseilabschnitt des Zugseils (20) vollständig umgibt.

11. Schultergelenkorthese (10) nach einem der vorangehenden Ansprüche, **dadurch gekennzeichnet, dass** die Armbandage (12) zwei Öffnungen zum Durchführen eines Armes des Patienten umfasst oder/und wenigstens ein Verschlusselement aufweist, das dazu ausgelegt ist, die Armbandage (12) an einem Arm des Patienten zu fixieren.

12. Schultergelenkorthese (10) nach einem der vorhergehenden Ansprüche, **dadurch gekennzeichnet, dass** die Schulterbandage (14) dazu ausgelegt ist, im Tragezustand der Schultergelenkorthese (10) die der zu behandelnden Schulter benachbarte Brust sowie den benachbarten Achselbereich des Patienten zumindest teilweise zu bedecken und unter der kontralateralen Achsel des Patienten durchgeführt zu werden.

13. Schultergelenkorthese (10) nach einem der vorhergehenden Ansprüche, **dadurch gekennzeichnet, dass** das Seilzugsystem (18) genau ein Zugseil (20) umfasst, das offen oder geschlossen ist.

## Claims

1. A shoulder orthosis (10) for stabilizing the shoulder of a human patient, comprising:
- an arm brace (12) for attachment to an arm of the patient such that the arm brace (12) at least partially surrounds the patient's upper arm when the shoulder orthosis is worn,
- a shoulder brace (14) for attachment to a shoulder of the patient such that the shoulder brace (14) at least partially covers the patient's shoulder when the shoulder orthosis is worn, and
- a pulley system (18) connecting the arm brace (12) to the shoulder brace (14) via a deflected pull cord (20),
wherein the pulley system (18) comprises at least one cord deflection member (22) and at least one cord tensioning member (24), the pull cord (20) extending on both sides of the cord deflection member (22) with two pull cord portions (34) and having a tensile effect, and the pull cord (20) being adjustable in its length having a tensile effect with the cord tensioning member (24) such that the positioning of the arm brace (12) relative to the shoulder brace (14) is adjustable,
wherein
the cord deflection member (22) is fastened to the shoulder brace (14) such that when the shoulder orthosis (10) is worn, the cord deflection member (22) is arranged on the patient's shoulder,
wherein
- at least one further cord deflection member (36, 38) is attached to the arm brace (12), the pull cord (20) passing through the at least one further cord deflection member (36, 38) starting from the shoulder brace (14) such that a further pull cord portion (40) connects the arm brace (12) to the shoulder brace (14) via the at least one further cord deflection member (36, 38), **characterized in that**
- the at least one further cord deflection member (36, 38) arranged on the arm brace (12) can be removed from the arm brace (12) without being damaged,
or that
the pull cord (20) can be disengaged from the at least one further cord deflection member (36, 38) arranged on the arm brace (12).

2. The shoulder orthosis (10) of claim 1, **characterized in that** the at least one further cord deflection member (36, 38) arranged on the arm brace comprises two cord deflection members (36, 38), a first one of the two cord deflection members (36) being arranged on the front side of the arm brace (12) when the shoulder orthosis (10) is worn, and a second one of the two cord deflection members (38) being arranged on the rear side of the arm brace (12) when the shoulder orthosis (10) is worn.

3. The shoulder orthosis (10) of any one of the preceding claims, **characterized in that** the shoulder brace (14) comprises at least one further cord deflection member (44) on its front side, which, when the shoulder orthosis is worn, is arranged on the patient's chest below the collarbone (30).

4. The shoulder orthosis (10) of any one of the preceding claims, **characterized in that** the shoulder brace (14) comprises at least one further cord deflection member (46, 48) on its rear side, which, when the shoulder orthosis is worn, is arranged in the area of the patient's shoulder blade (56) or humeral head (60), the shoulder brace (14) preferably comprising two cord deflection members (46, 48) on its rear side, one being arranged on the patient's humeral head (60) and one being arranged closer to the patient's spine.

5. The shoulder orthosis (10) of any one of the preceding claims, **characterized in that** the cord deflection member (22) which, when the shoulder orthosis is worn, is arranged on the patient's shoulder is connected to the shoulder brace (14) by means of a connecting member (77) allowing, preferably elastic, movement of the pull cord (20) relative to the shoulder brace (14).

6. The shoulder orthosis (10) of any one of the preceding claims, **characterized in that** when the shoulder orthosis (10) is worn, the cord tensioning member (24) is arranged on the shoulder brace (14) close to the sternum.

7. The shoulder orthosis (10) of any one of the preceding claims, **characterized in that** the cord tensioning member (24) changes the length of the pull cord (20) having a tensile effect through translational or rotational motion of the pull cord (20).

8. The shoulder orthosis (10) of any one of the preceding claims, **characterized in that** the pull cord (20) runs within a pull cord guide (86-92) connected to the shoulder brace (14) at least in sections or/and in at least one part of the cord deflection members (22, 36, 38, 44, 46, 48).

9. The shoulder orthosis (10) of any one of the preceding claims, **characterized in that** a supporting material (16) of the shoulder brace (14) or/and the arm brace (12) is a flexible, elastic fabric material, wherein a reinforcing material (74), e.g. containing plastic, is preferably arranged between the supporting material (16) of the shoulder brace (14) or/and the arm brace (12) and at least one of the cord deflection members (22, 36, 38, 44, 46, 48).

10. The shoulder orthosis (10) of any one of the preceding claims, wherein one of the cord deflection members (22, 36, 38,44, 46, 48) fully surrounds at least one corresponding pull cord portion of the pull cord (20).

11. The shoulder orthosis (10) of any one of the preceding claims, **characterized in that** the arm brace (12) comprises two openings for passing through an arm of the patient or/and at least one locking member configured to secure the arm brace (12) on an arm of the patient.

12. The shoulder orthosis (10) of any one of the preceding claims, **characterized in that** the shoulder brace (14), when the shoulder orthosis (10) is worn, is configured to at least partially cover the patient's chest adjacent to the shoulder to be treated as well as adjacent armpit area, and to be passed below the patient's contralateral armpit.

13. The shoulder orthosis (10) of any one of the preceding claims, **characterized in that** the pulley system (18) comprises exactly one pull cord (20) that is open or closed.

## Revendications

1. Orthèse d'articulation d'épaule (10) pour stabiliser l'épaule d'un patient humain, comprenant :
- un bandage de bras (12) destiné à être appliqué sur un bras du patient, de sorte que le bandage de bras (12) entoure au moins partiellement le bras du patient à l'état porté de l'orthèse d'articulation d'épaule,
- un bandage d'épaule (14) destiné à être appliqué sur une épaule du patient, de sorte que le bandage d'épaule (14) recouvre au moins partiellement l'épaule du patient à l'état porté de l'orthèse d'articulation d'épaule, et
- un système de traction à câble (18) qui relie le bandage de bras (12) au bandage d'épaule (14) par l'intermédiaire d'un câble de traction renvoyé (20),
dans laquelle le système de traction à câble (18) comprend au moins un élément de renvoi de câble (22) et au moins un élément de tension de câble (24), le câble de traction (20) s'étendant des deux côtés de l'élément de renvoi de câble (22) avec deux tronçons de câble de traction (34) agissant en traction et le câble de traction (20) pouvant être modifié dans sa longueur agissant en traction avec l'élément de tension de câble (24), de telle sorte que le positionnement du bandage de bras (12) par rapport au bandage d'épaule (14) est réglable,
dans laquelle l'élément de renvoi de câble (22) est fixé au bandage d'épaule (14) de telle sorte que, à l'état porté de l'orthèse d'articulation d'épaule (10), l'élément de renvoi de câble (22) est disposé sur l'épaule du patient,
dans laquelle au moins un autre élément de renvoi de câble (36, 38) est monté sur le bandage de bras (12), le câble de traction (20) étant guidé à travers ledit au moins un autre élément de renvoi de câble (36, 38) en partant du bandage d'épaule (14), de sorte qu'un autre tronçon de câble de traction (40) relie le bandage de bras (12) au bandage d'épaule (14) par l'intermédiaire dudit au moins un autre élément de renvoi de câble (36, 38),
**caractérisée en ce que**
ledit au moins un autre élément de renvoi de câble (36, 38) qui est disposé sur le bandage de bras (12) peut être retiré du bandage de bras (12) sans être détruit, ou **en ce que**
le câble de traction (20) peut être amené hors de prise avec ledit au moins un autre élément de renvoi de câble (36, 38) qui est disposé sur le bandage de bras (12).

2. Orthèse d'articulation d'épaule (10) selon la revendication 1, **caractérisée en ce que** ledit au moins un autre élément de renvoi de câble (36, 38) qui est disposé sur le bandage de bras est constitué de deux éléments de renvoi de câble (36, 38), un premier des deux éléments de renvoi de câble (36) étant disposé sur le côté avant du bandage de bras (12) à l'état porté de l'orthèse d'articulation d'épaule (10) et un deuxième des deux éléments de renvoi de câble (38) étant disposé sur le côté arrière du bandage de bras (12) à l'état porté de l'orthèse d'articulation d'épaule (10).

3. Orthèse d'articulation d'épaule (10) selon l'une des revendications précédentes, **caractérisée en ce que** le bandage d'épaule (14) comprend sur son côté avant au moins un autre élément de renvoi de câble (44) qui, à l'état porté, est disposé du côté de la poitrine en dessous de la clavicule (30) du patient.

4. Orthèse d'articulation d'épaule (10) selon l'une des revendications précédentes, **caractérisée en ce que** le bandage d'épaule (14) comprend sur son côté arrière au moins un autre élément de renvoi de câble (46, 48) qui, à l'état porté, est disposé dans la zone de l'omoplate (56) ou de la tête d'humérus (60) du patient, le bandage d'épaule (14) comprenant sur son côté arrière de préférence deux éléments de renvoi de câble (46, 48), l'un étant disposé plus près de la tête d'humérus (60) du patient et l'autre plus près de la colonne vertébrale du patient.

5. Orthèse d'articulation d'épaule (10) selon l'une des revendications précédentes, **caractérisée en ce que** l'élément de renvoi de câble (22) disposé sur l'épaule du patient à l'état porté est relié au bandage d'épaule (14) au moyen d'un élément de liaison (77) qui autorise un mouvement, de préférence élastique, du câble de traction (20) par rapport au bandage d'épaule (14).

6. Orthèse d'articulation d'épaule (10) selon l'une des revendications précédentes, **caractérisée en ce que** l'élément de tension de câble (24) est disposé à proximité du sternum sur le bandage d'épaule (14) à l'état porté de l'orthèse d'articulation d'épaule (10).

7. Orthèse d'articulation d'épaule (10) selon l'une des revendications précédentes, **caractérisée en ce que** l'élément de tension de câble (24) modifie la longueur du câble de traction (20) agissant en traction au moyen d'un mouvement de translation ou de rotation du câble de traction (20).

8. Orthèse d'articulation d'épaule (10) selon l'une des revendications précédentes, **caractérisée en ce que** le câble de traction (20) s'étend au moins sur certaines parties ou/et pour au moins une partie des éléments de renvoi de câble (22, 36, 38, 44, 46, 48) à l'intérieur d'un guide de câble de traction (86-92) qui est relié au bandage d'épaule (14).

9. Orthèse d'articulation d'épaule (10) selon l'une des revendications précédentes, **caractérisée en ce qu'**un matériau de support (16) du bandage d'épaule (14) ou/et du bandage du bras (12) est un matériau en tissu flexible et élastique, un matériau de renforcement (74), par exemple en matière plastique, étant de préférence disposé entre le matériau de support (16) du bandage d'épaule (14) ou/et du bandage du bras (12) et au moins l'un des éléments de renvoi de câble (22, 36, 38, 44, 46, 48).

10. Orthèse d'articulation d'épaule (10) selon l'une des revendications précédentes, dans laquelle au moins l'un des éléments de renvoi de câble (22, 36, 38, 44, 46, 48) entoure complètement au moins un tronçon de câble de traction correspondant du câble de traction (20).

11. Orthèse d'articulation d'épaule (10) selon l'une des revendications précédentes, **caractérisée en ce que** le bandage de bras (12) comprend deux ouvertures pour le passage d'un bras du patient ou/et comprend au moins un élément de fermeture qui est conçu pour fixer le bandage de bras (12) à un bras du patient.

12. Orthèse d'articulation d'épaule (10) selon l'une des revendications précédentes, **caractérisée en ce que** le bandage d'épaule (14) est conçu pour, à l'état porté de l'orthèse d'articulation d'épaule (10), recouvrir au moins partiellement le thorax adjacent à l'épaule à traiter ainsi que la zone d'aisselle adjacente du patient et pour être passé sous l'aisselle controlatérale du patient.

13. Orthèse d'articulation d'épaule (10) selon l'une des revendications précédentes, **caractérisée en ce que** le système de traction à câble (18) comprend exactement un câble de traction (20) qui est ouvert ou fermé.
